(19) Europäisches Patentamt
European Patent Office
Office européen des brevets

(11) **EP 2 323 541 B1**

(12) **EUROPÄISCHE PATENTSCHRIFT**

(45) Veröffentlichungstag und Bekanntmachung des Hinweises auf die Patenterteilung:
**28.03.2012 Patentblatt 2012/13**

(21) Anmeldenummer: **10707531.9**

(22) Anmeldetag: **09.03.2010**

(51) Int Cl.:
*A61B 1/04* (2006.01)    *A61B 1/05* (2006.01)

(86) Internationale Anmeldenummer:
**PCT/EP2010/052991**

(87) Internationale Veröffentlichungsnummer:
**WO 2010/105946 (23.09.2010 Gazette 2010/38)**

(54) **EIN ENDOSKOP UND EINE BILDAUFNAHMEVORRICHTUNG**

ENDOSCOPE AND IMAGING DEVICE

ENDOSCOPE ET DISPOSITIF DE PRISE DE VUES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **17.03.2009 DE 102009013761**

(43) Veröffentlichungstag der Anmeldung:
**25.05.2011 Patentblatt 2011/21**

(73) Patentinhaber: **Fraunhofer-Gesellschaft zur Förderung der angewandten Forschung e.V.**
**80686 München (DE)**

(72) Erfinder:
• **GUTIÉRREZ BORONAT, Javier**
  **91088 Bubenreuth (DE)**
• **JAHN, Jasper**
  **90489 Nürnberg (DE)**
• **SCHNEIDER, Armin**
  **80333 München (DE)**
• **HOELLER, Kurt**
  **91054 Erlangen (DE)**

(74) Vertreter: **Schenk, Markus et al**
**Schoppe, Zimmermann, Stöckeler & Zinkler**
**Patentanwälte**
**Postfach 246**
**82043 Pullach bei München (DE)**

(56) Entgegenhaltungen:
US-A1- 2005 020 883    US-A1- 2005 154 260
US-A1- 2008 108 870    US-A1- 2008 159 653

**Beschreibung**

Die vorliegende Erfindung bezieht sich auf ein Endoskop und auf eine Bildaufnahmevorrichtung und insbesondere auf eine CCD-Chip-nahe integrierte Inertialsensorik zur Bildrotationskorrektur, insbesondere in der flexiblen und drahtlosen Endoskopie.

Ein Inertialsensor ist beispielsweise in der Lage, die auf den Sensor einwirkende Beschleunigung oder Drehung (beispielsweise bezüglich des Gravitationsfeldes) zu messen. Inertialsensoren sind meist sensitiv in bezug auf eine der drei Raumrichtungen oder Drehrichtungen um eine der drei Raumrichtungen, so dass über die Zerlegung der Erdbeschleunigung auf die drei Raumrichtungen die aktuelle Orientierung zum Erdmittelpunkt angegeben werden kann. Inertialsensoren können beispielsweise in Form von mikroelektromechanischen Systemen (MEMS) gefertigt sein, so dass der Platzbedarf im Vergleich zu herkömmlichen Beschleunigungsmessern deutlich niedriger ist.

Endoskope dienen der Erfassung optischer Bilder und weisen ein distales Ende (=optischer Eingang) und ein proximales Ende auf. Bei flexiblen Endoskopen sind die Orientierungen (=räumliche Ausrichtungen) des distalen und proximalen Endes weitestgehend unabhängig voneinander, währenddessen bei starren Endoskopen (Laparoskope) eine feste Beziehung zwischen den Orientierungen der beiden Endoskopenden besteht.

Die translumenale endoskopische Chirurgie unter Nutzung natürlicher Öffnungen ist zu einer der größten Herausforderungen der chirurgischen Prozeduren geworden und besitzt das Potenzial die minimal invasive Chirurgie (MIS = Minimale Invasive Surgery) zu verdrängen. Zur Zeit wird die minimal invasive Chirurgie im wesentlichen von Chirurgen unter Nutzung starrer Laparoskope durchgeführt, wobei Laparoskope beispielsweise in dem Bauchraum von außen eingeführt werden. Andererseits verwenden Gastroenterologen flexible Videoendoskope, um beispielsweise Läsionen in dem gastrodigestiven Trakt (Speiseröhre, Magen, Darm, etc.) zu erkennen und zu entfernen. Aber auch die translumenale endoskopische Chirurgie erfordert flexible Endoskope, um in die Bauchhöhle vorzudringen und chirurgische Instrumente und Hilfsmittel einzuführen, so dass Eingriffe durchgeführt werden können.

Gastroenterologen sind geübt und ausgebildet, um durch Hohlräume wie beispielsweise den Darm, Magen oder die Speiseröhre zu navigieren, indem ein flexibles Videoendoskop gedreht, gedrückt oder gezogen wird und zwar unabhängig von der Orientierung, der Drehung und des Neigungswinkels der Endoskopspitze und der Bildorientierung, die auf dem Monitor dargestellt wird. Chirurgen sind andererseits am ehesten mit starren Endoskopen vertraut, bei denen eine feste Beziehung zwischen der Endoskopspitze und dem Körperinneren des Patienten besteht, da dessen Position sich nicht während des Eingriffs ändert.

Um Chirurgen bei der Interpretation und beim Erkennen von Bildern von flexiblen Videoendoskopen zu helfen, ist es deshalb erforderlich, automatisch das Bild zu korrigieren und bezüglich einer fest definierten Hauptachse umzuorientieren. Das Problem der "verdrehten Bilder" ist in hybriden Endoskopuntersuchungen sogar noch bedeutsamer, da in diesem Fall zusätzlich zu den Endoskopen noch Mikroinstrumente durch die Bauchwand eingeführt werden, so dass der Innenraum einerseits dargestellt wird und andererseits Aufgaben im Rahmen extrem komplexer Eingriffe durchgeführt werden.

Das zu lösende Problem kann auch wie folgt umschrieben werden. In der Medizin werden in der Gastroenterologie und in der minimal invasiven Chirurgie Videobilder zur Durchführung von diagnostischen Untersuchungen und operativen Eingriffen eingesetzt. Dazu stehen starre und flexible Endoskope sowie drahtlose Endoskopiekapseln zur Verfügung. Während der Arzt bei starren Endoskopen (Laparoskope) mit der führenden Hand noch einen direkt Bezug zur Orientierung des Bildes hat, ist dies bei flexiblen Endoskopen und Endoskopiekapseln nicht mehr gegeben. Die Orientierung innerhalb des Körpers ohne Kenntnis der Orientierung des Kamerachips bedarf der ausgezeichneten Kenntnis der sichtbaren anatomischen Strukturen und einer hervorragenden dreidimensionalen Vorstellungskraft. Da hierfür entsprechende Erfahrung und Übung notwendig ist, stellt diese fehlende Information eine besondere Hürde bei interdisziplinären Eingriffe dar, bei denen z.B. ein Chirurg, der gewöhnlich mit Laparoskopen operiert, für bestimmte translumenale Zugänge ein flexibles Endoskop nutzen muss.

Intraoperativ können dreidimensionale Daten in Echtzeit z.B. durch sogenannte Time-of-Flight-Kameras, die keine Datennachbehandlung erfordern, erhalten werden, wobei Bilder mit einem Takt von mehr als 30 Hz erzeugt werden können. Die Echtzeitberechnung von Anpassungsparametern stellt jedoch immer noch eine Herausforderung dar.

Bisher wurde die Orientierung endoskopischer Bilder mittels sogenannter elektromagnetischer Tracker realisiert. Dazu muss jedoch ein externes Feld aufgebaut werden, das durch der Einsatz metallischer Instrumente gestört werden würde. Dies hat entsprechende Einschränkungen zur Folge.

Eine weitere Möglichkeit ist die Gewinnung von intraoperativen dreidimensionalen Daten mittels Verfahren wie Shape-from-Shading, Structure-from-Motion, Stereobild-Triangulation, Beleuchtung mit strukturiertem Licht oder Time-of-Flight-Sensoren. Die so gewonnenen dreidimensionalen Daten werden mit präoperativen Computertomographen oder mit Magnetresonanztomographie-Datensätzen registriert (oder angepasst). Die entsprechenden Transformationsparameter können auch zur Rotationskorrektur verwendet werden. Der rechnerische Aufwand dafür ist allerdings so groß, dass derzeit überhaupt keine Anwendungen in Echtzeit möglich sind. Auch in mittelbarer Zukunft wird es aufgrund der riesigen zu verarbeitenden Datenmengen und Vorbedingungen kein Regelszenario sein.

Der Einsatz von Inertialsensorik in der Endoskopie wurde bereits in US 7211042 B2 beschrieben, wobei das dort beschriebene Verfahren ein Laparoskop nutzt. Da das Problem der fehlenden Orientierung in der starren Laparoskopie aber deutlich geringer ist als in der flexiblen Endoskopie, hat die Bildrotationskorrektur eine geringere Bedeutung. Z.B. können bei den starren Endoskopen, die Erfassung von Drehrichtungen durch Inertialsensoren außerhalb des Körpers geschehen, so dass sich die Frage der Übertragung der Sensorsignale gar nicht oder kaum stellt. Bei flexiblen Endoskopen andererseits ist die Orientierung des distalen Endes des Endoskops im Allgemeinen vollständig unabhängig von der Orientierung des proximalen Endes und wenn am distalen Ende die Bilderfassung durchgeführt wird oder durchgeführt werden soll, stellt sich das oben beschriebene Problem, wie die Orientierung korrigiert bzw. festgestellt werden kann.

Dokument US2008/0159653 A1 beschreibt ein Endoskop laut Oberbegriff von Anspruch 1, eine Bildaufnahmevorrichtung laut Oberbegriff von Anspruch 11 und ein Verfahren laut Oberbegriff von Anspruch 14. Dokument US2005/0020883 A1 offenbart ein Endoskop und Verfahren wobei mittels Inertialsensoren die Verkippung eines Bildsensors erfasst wird um eine Bildkorrektur in Bezug auf die Orientierung des distalen Endes zu ermöglichen.

Ausgehend von diesem Stand der Technik, liegt der vorliegenden Erfindung die Aufgabe zugrunde, ein Endoskop zu schaffen, das eine Bilderfassung am distalen Ende erlaubt und gleichzeitig unabhängig von der Orientierung des proximalen Endes eine Bildkorrektur in Bezug auf die Orientierung des distalen Endes ermöglicht. Es soll ferner eine Bildaufnahmevorrichtung geschaffen werden, die es ermöglicht, Daten, die die Orientierung des Bildsensors am distalen Ende beschreiben, möglichst effizient an das proximale Ende zu übertragen.

Diese Aufgabe wird durch ein Endoskop nach Anspruch 1, eine Bildaufnahmevorrichtung nach Anspruch 11 und ein Verfahren zur Aufnahme eines Bildes nach Anspruch 14 gelöst.

Der Kerngedanke der vorliegenden Erfindung besteht darin, ein Endoskop zu schaffen, bei dem ein Bildsensor und ein Inertialsensor an einem distalen Ende des Endoskops angeordnet sind, wobei der Inertialsensor eine Verkippung des Bildsensors relativ zum Gravitationsfeld erfasst und ein Verkippungssignal als Maß der Verkippung ausgibt. Der Inertialsensor misst dazu beispielsweise eine Beschleunigung oder eine Drehung relativ zu dem Schwerefeld des Gravitationsfelds, so dass jede Drehung (=Verkippung) um eine der drei Raumachsen erfassbar ist. Die Erfindung betrifft ebenfalls ein Verfahren zur Übertragung des Verkippungssignals. Dabei wird das Verkippungssignals und die Bilddaten zu einem erweiterten Datenstrom kombiniert, wobei ein Teil der Bilddaten durch das Verkippungssignal ersetzt werden.

Ausführungsbeispiele schaffen somit ein Endoskop mit einem Bildsensor und einem Inertialsensor am distalen Ende desselben, wobei der Inertialsensor ausgebildet ist, um eine Verkippung des Bildsensors relativ zum Gravitationsfeld zu erfassen. Weitere Ausführungsbeispiele umfassen eine Bildaufnahmevorrichtung mit einem digitalen Bildsensor zum Erzeugen von Bilddaten, einem Inertialsensor zur Erfassung einer Verkippung relativ zum Gravitationsfeld, um ein Verkippungssignal zu erhalten. Die Bildaufnahmevorrichtung weist ferner eine Steuereinrichtung zum Kombinieren des Verkippungssignals und der Bilddaten zu einem erweiterten Datenstrom unter Ersetzung von Teilen der Bilddaten durch das Verkippungssignal auf. Ausführungsbeispiele umfassen ebenfalls eine Endoskopkapsel mit einer solchen Bildaufnahmevorrichtung und ein Verfahren zum Übertragen von Endoskopbilder. Ausführungsbeispiele umfassen ebenso ein Verfahren zur Aufnahme eines Bildes. Das Verfahren umfasst die Schritte des Erzeugens von Bilddaten durch einen Bildsensor, des Erfassens einer Verkippung relativ zum Gravitationsfeld durch einen Inertialsensor und Bereitstellen eines Verkippungssignals und den Schritt des Kombinierens des Verkippungssignals mit den Bilddaten zu einem erweiterten Datenstrom unter Ersetzung von Teilen der Bilddaten durch das Verkippungssignal.

Ein wichtiger Schritt zur Lösung der eingangs beschriebenen Probleme ist die Platzierung des Inertialsensors in der unmittelbaren Nähe des Bildsensors - noch besser eine Integration auf demselben Chip und zwar am distalen Ende des Endoskops. Es ist somit keine optische Übertragung des Bildes erforderlich - eine Optik kann optional lediglich genutzt werden, um das Bild auf den Bildsensor zu fokussieren. Die Übertragung des Bildes kann beispielsweise rein elektronisch in Form eines digitalen Datenstroms erfolgen.

Die Integration von Inertialsensoren nahe dem Bildsensor ist Voraussetzung für einen Einsatz in der flexiblen und Kapselendoskopie. Zusätzlich erforderliche Leitungen zur Übertragung des Inertialsensorsignals (Verkippungssignal) sind nur schwer in den äußerst beschränkten Raum flexibler Endoskope zu integrieren und ein zusätzlicher drahtloser Transmitter dürfte wiederum in der Endoskopiespitze bzw. Kapsel keinen Platz finden. Daher nutzen Ausführungsbeispiele der vorliegenden Erfindung für die Übertragung der Inertialsensordaten den Bilddatenstrom und integrieren das Verkippungssignal in demselben. Dadurch wird die gewünschte Unterbringung des Inertialsensors nahe am Bildsensor möglich. Diese Realisierung trägt dem begrenzten Raumangebot, da neben der Bildübertragung auch Arbeitskanäle für invasive Eingriffe benötigt werden, bei flexiblen Endoskope Rechnung. Behelfsweise ließe sich zwar ein Arbeitskanal nutzen, dieser Arbeitskanal würde jedoch dann komplett blockiert sein. Bei der Kapsel ist eine drahtgebundene Übertragung ohnehin gar nicht möglich.

Bei weiteren Ausführungsbeispielen werden als Bildsensor beispielsweise CCD-Chips (CCD = charge-coupled device) mit einer entsprechenden Optik direkt an der Endoskopspitze integriert. Von dort wird das Signal zum Endoskopende hin digital übertragen, wo es dann digital abgegriffen werden kann. Um die besagte zusätzliche Leitung für den MEMS-Sensor (Inertialsensor) zu vermeiden, soll erfindungsgemäß das MEMS-Signal (Verkippungssignal) mit dem digitalen Video-Signal verknüpft und über dieselbe Leitung übertragen werden.

Gemäß Ausführungsbeispielen wird das Bild in drei Farbkanälen als RGB-Signal mit 8 Bits pro Kanal codiert. Der Bildsensor kann beispielsweise eine CCD-Kamera mit einem CCD-Chip von m x n Pixel (m = Spaltenanzahl, n = Zeilenanzahl) umfassen. Das Verkippungssignal kann ebenfalls mit 8 Bits pro Verkippung (für jede der drei Raumrichtungen = MEMS-Achsen) codiert werden. Ausführungsbeispiele nutzen zum Kombinieren des Verkippungssignals mit dem Datenstrom beispielsweise die folgenden drei Möglichkeiten:

1. Anstelle der Bilddaten für einen Pixel (z.B. 3-Byte bei einer 8-Bit-Codierung der drei Farbkanäle RGB) in einer der Chipecken (z.B. der Pixel in der ersten Zeile und ersten Spalte) werden die drei Verkippungswerte für die MEMS-Achsen x, y und z codiert und übertragen. Dazu werden Daten, die ein Pixel im Bild kodieren, ersetzt. Beispielsweise werden vom CCD-Controller anstelle der Ladungen des zu ersetzenden Pixels und neben den Ladungen aller übrigen m*n-1 RGB-Pixel die drei Spannungen für die drei Achsen eines analogen MEMS-Sensors abgegriffen und anschließend analog/digital gewandelt. Das kann zum Beispiel zusammen mit den Ladungen der verbleibende Pixel geschehen. Dazu kann es erforderlich sein, beispielsweise über ein Widerstandsnetzwerk eine Anpassung der Ladungen/Spannungen auf den Wertebereich der CCD-Ladungen vorzunehmen. Übertragen wird dann wieder ein ganzes Bild, allerdings sollte bei der Visualisierung die MEMS-Information extrahiert und der gewählte Pixel wieder interpoliert werden.

2. Alternativ kann die unter 1. beschriebene Methode dahin gehend modifiziert werden, dass auch ein digitales 3-Byte-MEMS-Signal vom CCD-Controller/Encoder eingelesen und anstelle des entsprechenden RGB-Pixels codiert und übertragen wird. Hierbei wird das Verkippungssignal zunächst digitalisiert und nicht analog in dem CCD-Controller eingegeben wie unter Punkt 1.

3. Die Variante unter 2. kann noch dahin gehend verbessert werden, dass die digital eingelesenen MEMS-Werte unter Substitution nur bestimmte Bits jedes Kanals (r/g/b) vorgenommen wird. Beispielsweise können die sogenannten Least Significant Bits von 256 Pixel bei einem 8-bit Verkippungssignal ersetzt werden. Für alle drei Verkippungssignale wären dann beispielsweise 3*256 Pixel erforderlich. Diese Daten können beispielsweise in Pixel im Randbereich des Bildes codiert und übertragen werden - z.B. können dazu Pixel in der ersten Zeile oder auch in der ersten Spalte genutzt werden.

Somit kann die Übertragung des digitalen Inertialsensorsignals statt über separate Leitungen wie beschrieben durch eine Einbettung in das Signal des Bildsensors erfolgen. Die Einbettung in den bereits vorhandenen Datenstrom bedeutet für eine Anwendung im Bereich der flexiblen Endoskopie und der drahtlosen Kapselendoskopie eine massive Erleichterung der Umsetzbarkeit, da vorhandene Ressourcen effizient genutzt werden.

Ferner können somit MEMS-basierte tri-axiale Inertialsensoren zur Bildstabilisierung aufgrund ihrer geringen Baugröße auch bei platzkritischen Anwendungen in der flexiblen Endoskopie oder der Kapselendoskopie in unmittelbarer Nähe zum Bildsensor integriert werden. Über dem an den drei Achsen gemessenen Anteil der Schwerkraft kann auf die Orientierung des Bildsensors bezüglich des Erdmittelpunkts geschlossen werden. Das aufgenommene Bild bzw. der aufgenommene Videostrom kann digital um diesen Drehwinkel wieder zurückgedreht werden, was einen stabilen Horizont sowie eine Übereinstimmung von realen und angezeigten Neigungen bzw. Orientierungen aufgenommener Objekte und Bewegungen ermöglicht. Somit ist es möglich ein stabiles Bild auf dem Bildschirm zu erhalten, selbst dann, wenn das Endoskop bewegt wird (z.B. gedreht oder verschoben wird). Optional kann parallel auch das Originalbild mit angezeigt werden, um gleichzeitig ein weitere Kontrolle zur Sicherheit zu haben.

Neben der medizinischen Gastroenterologie und der minimal invasiven Endoskopie sowie deren Kombination, wie beispielsweise die sogenannte Natural Orifice Translumenal Endoscopic Surgery (NOTES) ist eine Anwendung auch im industriellen Bereich zur Qualitätssicherung denkbar. Industrielle Anwendungen umfassen beispielsweise Untersuchungen für Kanäle oder versteckte Hohlräume, die durch andere Mittel nicht einsehbar sind.

Ein weiterer Vorteil von Ausführungsbeispielen besteht schließlich darin, dass durch eine Integration des Inertialsensors auf einen CCD-Chip eine weitere Miniaturisierung erreichbar ist. Ausführungsbeispiele sind somit ebenfalls für die Orientierungserkennung oder Bildstabilisierung von Digitalkameras einsetzbar. Ausführungsbeispiele der vorliegenden Erfindung werden nachfolgend Bezug nehmend auf die beiliegenden Zeichnungen näher erläutert. Es zeigen:

Fig. 1    eine schematische Darstellung einer Endoskopspitze gemäß Ausführungsbeispielen der vorliegenden Erfindung;

Fig. 2    eine schematische Darstellung eines flexiblen Endoskops mit den unterschiedlichen Orientierungen der Endoskopspitze; und

Fig. 3 ein Blockdiagramm zur Dreh-Korrektur mit einem Algorithmus gemäß Ausführungsbeispielen.

Bezüglich der nachfolgenden Beschreibung sollte beachten werden, dass bei den unterschiedlichen Ausführungsbeispielen gleiche oder gleichwirkende Funktionselemente gleiche Bezugzeichen aufweisen und somit die Beschreibung dieser Funktionselemente in den verschiedenen Ausführungsbeispielen untereinander austauschbar sind.

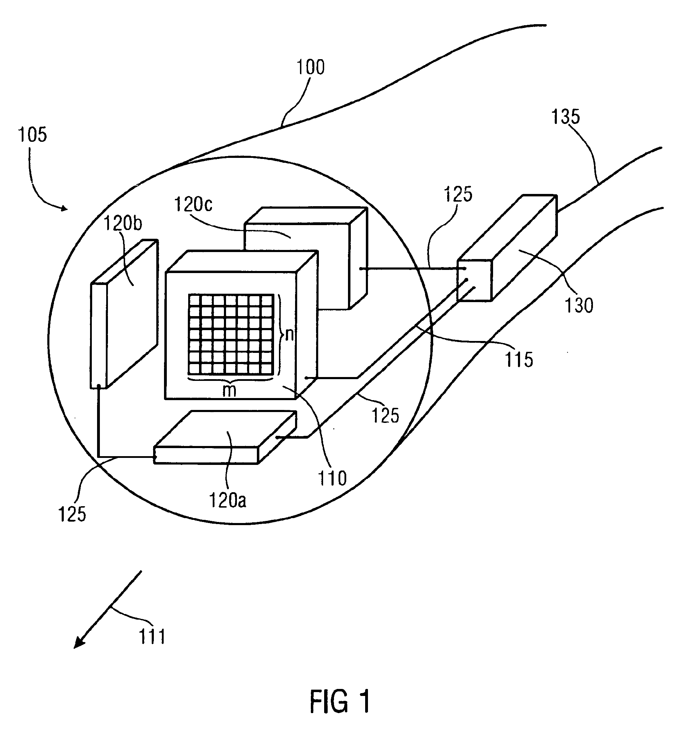

Fig. 1 zeigt beispielsweise ein distale Ende 105 eines flexiblen Endoskops 100 mit einem Bildsensor 110, drei Inertialsensoren 120a, 120b und 120c, die am distalen Ende des Endoskops 100 zusammen mit einer Steuereinrichtung 130 angeordnet sind. Das Endoskop 100 erfasst optische Bilder in eine Blickrichtung 111 auf die das distale Ende 105 gerichtet ist. Die drei Inertialsensoren 120 bilden beispielsweise ein orthogonales System, so dass Drehungen bezüglich der drei verschiedenen Raumrichtungen detektierbar sind. Die Inertialsensoren 120 erzeugen die entsprechenden Verkippungssignale, die über Signalleitungen 125 an die Steuereinrichtung 130 weitergeleitet werden. Der Bildsensor 110 erzeugt von einem Objekt (nicht gezeigt) in der Blickrichtung 111 Bilddaten, die über eine Signalleitung 115 zu der Steuereinrichtung 130 weitergeleitet werden. Die Steuereinheit 130 kombiniert die Bilddaten des Bildsensors 110 mit den Daten der Inertialsensoren 120 zu einem erweiterten Datenstrom, der über eine (elektrische) Übertragungsleitung 135 an das proximale Ende (in der Figur nicht gezeigt) weiterleitet wird.

Optional kann das distalen Ende 105 des Endoskops 100 Beleuchtungseinrichtungen (nicht in Fig. 1 gezeigt) aufweisen, um das Objekt zu beleuchten. Der Bildsensor 110 kann beispielsweise einen CCD-Chip aufweisen und die Erfassung der Bilder kann beispielsweise unter Nutzung des RGB-Farbschemas erfolgen. Der CCD-Chip ist ausgebildet das Bild als ein Array von Bildpixel zu erfassen, wobei der CCD-Chip jedem Pixel eine Ladungsmenge zuordnet, die proportional zur eingestrahlten Lichtmenge auf dem Bildpixel ist. Um Farbbilder zu erfassen weisen CCD-Chips Zellen auf, die verschiedene Sensitivitäten hinsichtlich einzelner Farbkomponenten (z.B. rot, grün und blau) aufweisen. Damit werden für jedem Pixel beispielsweise drei Komponentensignale (Kanäle) erfasst, deren Überlagerung den Farbpunkt wiedergeben. Dabei kann die Farbtiefe beispielsweise 8 Bit pro Kanal betragen, so dass jeder Pixel durch 3-Byte codiert wird. Der CCD-Chip kann beispielsweise eine Auflösung von m Spalten und n Zeilen aufweisen, so dass die Anzahl der Pixel durch m x n gegeben ist.

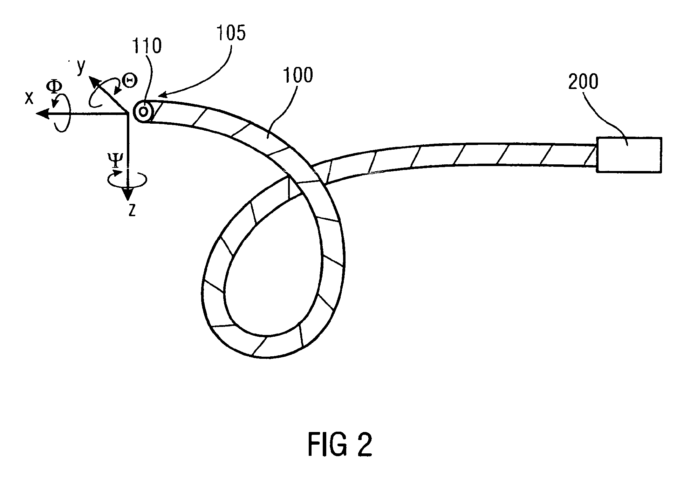

Fig. 2 zeigt das flexible Endoskop 100 mit dem Bildsensor 110 an dem distalen Ende 105 und einer Bildauswerteeinheit 200 am proximalen Ende, so dass zwischen dem distalen Ende 105 und dem proximalen Ende eine digitale Übertragung von Bilddaten entlang der Übertragungsleitung 135 erfolgt, da die optische Umwandlung des Bildes in digitale Bildinformationen bereits am distalen Ende 105 durchgeführt wird.

Um die Orientierung eines Endoskops relativ zu dem Gravitationsfeld zu beschreiben, wird im Allgemeinen ein kartesisches Endoskop-Navigationssystem mit den Achsen x, y und z als Referenzsystem genutzt. Die Blickrichtung 111 der Endoskopspitze 105 entspricht dabei der x-Richtung, senkrecht dazu zum Boden gerichtet (in Richtung der Schwerebeschleunigung) ist die z-Richtung und senkrecht zu beiden Richtungen parallel zur horizontalen Bildebene (z.B. nach rechts gerichtet) ist die y-Achse ausgerichtet. Drehungen um diesen Achsen werden wie folgt genannt: der Rollwinkel $\Phi$ beschreibt Drehungen um die x-Richtung, der Neigungswinkel $\theta$ beschreibt Drehungen um die y-Richtung und Drehungen um die z-Richtung werden durch den Ablenkwinkel $\Psi$ beschrieben. Die Gravitationsbeschleunigung wird als ein externer unabhängiger Vektor, der parallel zur z-Achse ist (so dass g die z-Komponente ist), betrachtet.

Wenn keine Bildkorrekturen bei Drehungen durchgeführt werden, dreht sich das Endoskopbild analog zu Drehungen des Endoskops. Da es keine explizite Winkelinformation gibt, kann zu der besagten Ausrichtung des Bildes lediglich der Einfluss der Gravitation bezüglich jeder der Achsen genutzt werden. Durch Drehungen wird die Richtung, aus der das Gravitationsfeld einwirkt, geändert. Um die raumartige Ausrichtung zu erhalten, kann diese Änderung durch die Rotationsparameter $\Phi$, $\theta$ und $\Psi$ in einem Inertialmesssystem (IMU = Inertial Measurement Unit) wie folgt parametrisiert werden (die Gravitation g als parallel zur z-Richtung angenommen wird):

$$\begin{pmatrix} F_x \\ F_y \\ F_z \end{pmatrix} = \begin{pmatrix} 1 & 0 & 0 \\ 0 & \cos(\Phi) & \sin(\Phi) \\ 0 & -\sin(\Phi) & \cos(\Phi) \end{pmatrix} \cdot \begin{pmatrix} \cos(\theta) & 0 & -\sin(\theta) \\ 0 & 1 & 0 \\ \sin(\theta) & 0 & \cos(\theta) \end{pmatrix} \cdot$$

$$(1)$$

$$\begin{pmatrix} \cos(\Psi) & \sin(\Psi) & 0 \\ -\sin(\Psi) & \cos(\Psi) & 0 \\ 0 & 0 & 1 \end{pmatrix} \cdot \begin{pmatrix} 0 \\ 0 \\ g \end{pmatrix} = \begin{pmatrix} -\sin(\theta)g \\ \sin(\Phi)\cos(\theta)g \\ \cos(\Phi)\cos(\theta)g \end{pmatrix}$$

wobei $F_{x,y,z}$ die gemessenen Beschleunigungen darstellen. Unter Verwendung der arctan-Funktion mit zwei Argumenten kann die Mehrdeutigkeit von $\pm$ n vermieden werden und man erhält für den Rollwinkel $\Phi$ und den Neigungswinkel $\theta$ die Werte:

$$\Phi = \arctan 2(F_y, F_z) \qquad , \tag{2}$$

$$\theta = \arcsin\left(\frac{-F_x}{g}\right) \tag{3}$$

wobei angenommen wird, dass $F_x \neq \pm g$. Mit Hilfe der Gravitationsbeschleunigung können allerdings nur zwei Freiheitsgrade bestimmt werden, so dass der Ablenkwinkel $\Psi$ durch dieses Verfahren nicht bestimmt wird. Für $F_x = \pm g$ (d.h. $\theta = \pm \Pi/2$ oder $\cos\theta = 0$ und somit $F_y = F_z = 0$) folgt ebenfalls dass der Rollwinkel $\Phi$ unbestimmt bleibt.

In der Auswerteeinheit 200 können verschiedene Korrekturen/Algorithmen ausgeführt werden, wobei im Folgenden ein Ausführungsbeispiel konkret beschrieben werden soll. Alternativ können diese Korrekturen/Algorithmen auch durch die Steuereinheit 130 ausgeführt werden.

Um negative Bewegungseinflüsse zu vermeiden, werden dazu zunächst Korrekturen lediglich berücksichtigt, wenn die Überlagerung der Beschleunigung zu der Gravitation unterhalb eines vorgegebenen Schwellwertes $\Delta F_{absmax}$ ist:

$$\left| \sqrt{F_x^2 + F_y^2 + F_z^2} - g \right| < \Delta F_{abs\,max} \qquad . \tag{4}$$

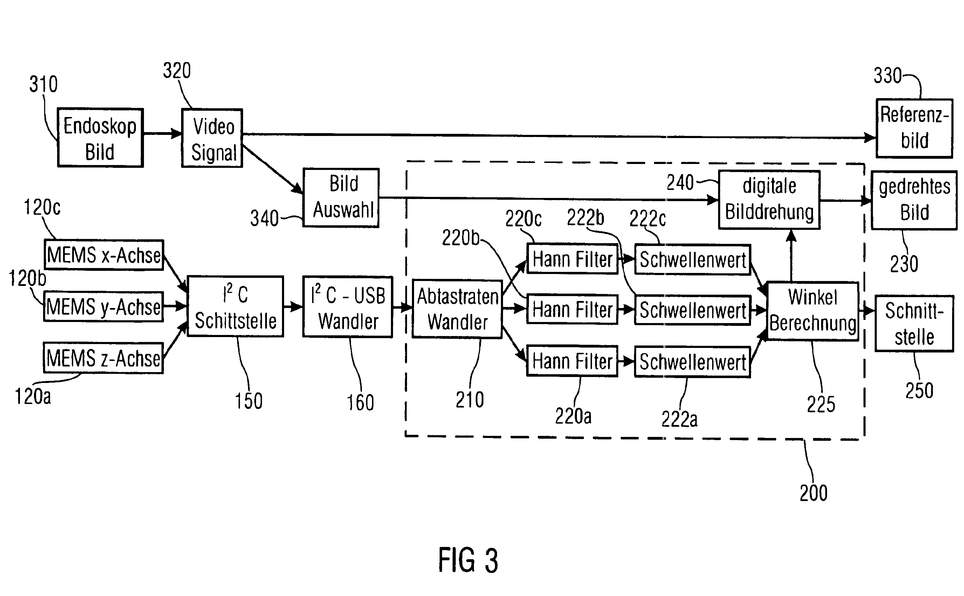

Fig. 3 zeigt ein Blockschaltbild für die Rotationskorrektur eines durch ein Endoskop 100 erhaltenen Bildes. Das Endoskopbild 310 wird dabei zunächst in ein Videosignal 320 umgewandelt, welches Komponenten, wie beispielsweise rot, grün und blau (ein sogenanntes RGB-Bild) umfassen kann. Das Videobild kann dann zunächst als Referenzbild 330 zur Verfügung gestellt. Gleichzeitig kann mittels einer Bildauswahl 340 (ein sogenannter Frame Grabber) ein Einzelbild aus dem Videosignal 320 extrahiert werden, welches dann innerhalb einer Bildauswerteeinheit 200 neu ausgerichtet oder gedreht wird. Es soll damit ein stabiler Horizont erreicht werden, so dass Drehungen der Endoskopspitze 105 das Bild kaum oder nicht drehen. Anschließend wird ein gedrehtes Bild 230 ausgegeben, welches mit dem ursprünglichen Referenzbild 330 (welches nicht korrigiert oder gedreht wurde) durch den Arzt z.B. verglichen werden kann.

Um die Drehung auszuführen, können beispielsweise als Inertialsensoren 120 MEMS-Inertialsensoren genutzt werden, z.B. einen ersten MEMS-Inertialsensor 120a für Drehungen um die z-Achse, einen zweiten MEMS-Inertialsensor 120b für Drehungen um die y-Achse und einen dritten MEMS-Inertialsensor 120c für Drehungen um die x-Achse. Die Inertialsensoren 120 erfassen dabei die Drehungen um die jeweilige Achse und geben die entsprechenden Winkelwerte (Verkippungswerte) aus, wobei die ausgegebenen Winkelwerte an eine Schnittstelle 150 weitergeleitet werden können. Die Schnittstelle kann beispielsweise Teil der Steuereinrichtung (130) sein und beispielsweise eine sogenannte 2-Wire $I^2C$-Schnittstelle umfassen, die ihrerseits die Winkelwerte der Inertialsensoren 120 kombiniert und an einen Wandler 160 (z.B. einen $I^2C$-zu-USB-Wandler), weitergibt. Das gewandelte Signal des Wandlers 160 kann dann an eine Bildauswerteeinheit 200 weitergeleitet werden, wo es beispielsweise zunächst durch einen Abtastratenwandler 210 modifiziert wird. Die Bildauswerteeinheit kann beispielsweise Teil der Steuereinrichtung 130 sein, kann aber auch räumlich davon getrennt sein.

Der Abtastratenwandler 210 kann beispielsweise die im folgenden beschriebene Anpassung der Abtastrate der Inertialsensoren 120 an die Bildabtastratenfrequenz durchführen. Konkret kann folgender Algorithmus genutzt werden. Zunächst wird durch eine Anfangsmessung eine vorhergehende 3x3 Kalibrierungsmatrix abgerufen, die Fehlausrichtungen und Skalierungsfehler berücksichtigt. Die Verkippungswerte können durch ein zeitliches Abtasten mit einer Abtastrate die deutlich oberhalb der Bildwiederholrate (Frame Rate) liegt, erhalten werden. Daher können im Rahmen eines Downsampling Extremwerte in einem nächsten Schritt eliminiert werden. Das Downsampling kann beispielsweise durch ein Mitteln erfolgen, beim dem 1 Sensorwerte $F_{xi}$, $F_{yi}$ und $F_{zi}$ (i=1,...,1) innerhalb eines Bildframes mit Gewichtungsfaktoren $w_i$ (mit einem maximalen Gewicht von 1/wo):

$$w_i \;=\; \frac{1}{\left| \sqrt{F_x^2 \;+\; F_y^2 \;+\; F_z^2} \;-\; g \right|}$$

multipliziert und anschließend aufsummiert werden. Danach wird die so erhaltene Summe durch die Summe aller Gewichtungsfaktoren $w_i$ normiert, so dass sich als Ergebnis der Mittelung die folgenden (gemittelten) Verkippungswerte ergeben:

$$\begin{pmatrix} F_x \\ F_y \\ F_z \end{pmatrix} = \sum_{i=1}^{n} \left( \begin{pmatrix} F_{xi} \\ F_{yi} \\ F_{zi} \end{pmatrix} \cdot w_i \right) \cdot \sum_{i=1}^{n} \left( w_i \right)^{-1}$$

Um ein sogenanntes Bouncing oder Jittering in dem Ergebnis der Winkelkorrektur zu vermeiden, wird ein zusätzliches Filtern genutzt. Beispielsweise können die erhaltenen Werte $F_{x,y,z}$ in einen Hann-Filter 220 gefiltert werden. Der Hann-Filter weist dabei wiederum einen ersten Hann-Filter 220a für eine Filterung des ersten Signals $F_z$ des ersten Inertialsensors 120a, einen zweiten Hann-Filter 220b für eine Filterung des zweiten Inertialsignals $F_y$ des zweiten Inertialsensors 120b und einen dritten Hann-Filter 220c für eine Filterung eines dritten Verkippungssignals $F_z$ des dritten Inertialsensors 120c auf.

Außerdem wird Schwellprozessoren 222 ein minimaler Variationsschwellwert $\Delta F_{axmin}$ genutzt, um ein schnelles Schwanken der Verkippungssignale zu unterdrücken. Solange wie die überlagerten Beschleunigungen, die durch die Gleichung (4) ausgerechnet wurden, unterhalb des Grenzwertes $\Delta F_{absmax}$ liegen, kann der Rollwinkel $\Phi$ und der Neigungswinkel $\theta$ durch die Gleichungen (2) und (3) berechnet werden. Wenn nicht, sind sie eingefroren bis zu dem Moment, wo $\Delta F_{absmax}$ wieder unterschritten wird. Diese Schwellwertbehandlung kann wiederum komponentenweise erfolgen. Konkret werden in einem ersten Schwellwertprozessor 222a lediglich Variationen oder Änderungen in dem ersten Verkippungssignal $F_z$ bearbeitet, die oberhalb des Schwellwerts liegen, so dass ein Rauschen unterdrückt werden kann. In ähnlicher Weise wird das zweite Verkippungssignal $F_y$ des zweiten Inertialsensors 120b nach der Übertragung und der Abtastratenwandelung 210 in einen zweiten Hann-Filter 220b gefiltert und anschließend in einem zweiten Schwellwertprozessor 222b eingelesen. Schließlich wird auch das dritte Verkippungssignal $F_x$ des dritten Inertialsensors 120c nach Übertragung der Abtastratenwandlung einem dritten Hann-Filter 220c zugeführt und nach Filterung einem dritten Schwellwertprozessor 222c zugeführt.

Die Schwellwertprozessoren 222 geben die Ausgangssignale an eine Einrichtung zur Winkelberechnung 225 aus, welche ausgebildet ist, um aus den Verkippungssignalen (nach Abtastratenwandelung, Hann-Filterung und Schwellwertvergleich) die Winkelwerte aus den Verkippungssignalen $F_{x,y,z}$ zu berechnen (z.B. unter Nutzung der Gleichungen (2) und (3)). Die berechneten Winkelwerte werden dabei zum einen an einer äußeren Schnittstelle 250 zur Verfügung gestellt und andererseits dazu benutzt, um in einer digitalen Bilddrehung 240 das ausgewählte Bild oder die Bildsequenz entsprechend zu drehen, so dass das gedrehte Bild 230 ebenfalls ausgegeben wird. Das gedrehte Bild kann an eine Anzeige (nicht gezeigt) angezeigt werden.

Wenn die Randwerte $\Delta F_{axmin}$ und $\Delta F_{absmax}$ geeignet gewählt sind, wird das Resultat kontinuierlich und zuverlässig sein, da nahezu alle überlagerten Bewegungen innerhalb einer gewöhnlichen Operation oder eines gewöhnlichen Eingriffes nicht zu diskontinuierlichen oder abrupten Winkeländerungen führen. Diese Randwerte können optional auch individuell angepasst werden. Beide Bilder sowohl das Referenzbild als auch das gedrehte Bild können angezeigt werden, so dass ein hohes Maß an Sicherheit sichergestellt ist.

Die Bilddrehung kann alternativ auch wie folgt beschrieben werden. Die Daten, die durch die Inertialsensoren 120 gemessen werden, werden als digitale Signale beispielsweise über eine sogenannte 2-Wire I$^2$C-Schnittstelle entlang des flexiblen Endoskops geleitet (d.h. vom distalen Ende mit Bildsensor und Inertialsensor zu dem proximalen Ende). Am proximalen Ende werden dann die Daten zur weiteren Prozessierung mit einem Adapter (z.B. I$^2$C-zu-USB-Adapter) gewandelt. Das Endoskopvideosignal kann seinerseits unter Nutzung eines externen USB-Videoaufnahmegeräts mit adäquater Auflösung eingelesen werden, so dass eine gewohnte Qualität für den Chirurgen zur Verfügung gestellt wird.

Die Drehung des Bildes kann beispielsweise mit Hilfe von zwei Schritten durchgeführt werden.

1. Es werden die Beschleunigungsdaten (Verkippungssignal) eingelesen und die Winkelberechnung kann gemäß den oben angegebenen Gleichungen bezüglich der drei Achsen durchgeführt werden.

2. Es wird ein Bild durch den Frame Grabber ausgewählt und anschließend mit Hilfe der berechneten Winkelwerte gedreht.

In klinischen Untersuchungen wurde während Tierversuchen die Navigation mittels eines hybriden starren Instruments unter Verwendung der NOTES-Prozedur mit einem definierten S-förmigen Zugang mit und ohne Bilddrehung durchgeführt. Die endoskopische Inertialmesseinrichtung 120 war an der Endoskopspitze eines flexiblen Endoskops 100 fixiert und während der Studie wurde das originale (nicht rotierte), analoge Videosignal auf einem Monitor dargestellt.

Beispielsweise können für die technische Durchführung folgende Daten verwendet werden. Die Verkippung oder Drehung um jede der Achsen, die durch den Inertialsensor detektiert wird, kann beispielsweise gleichmäßig mittels 8-Bit quantisiert werden und zwar in einer Region zwischen $\pm$ 2,3g. Dies bedeutet eine Quantisierungsgenauigkeit von 0,018g pro Schritt oder 110 Schritte für die ausgewählte Region von $\pm$ g. Dies ist hoch genug, um eine hinreichende Genauigkeit, sogar für relativ schwache oder leichte Bewegungen zu erreichen. Dies ist möglich, da der Rollwinkel $\Phi$ beispielsweise durch inverse trigonometrische Werte von zwei orthogonalen Verkippungen ($F_y$, $F_z$) erhalten wird. Einzelne, besonders starke Störungen der MEMS-Werte werden dabei durch kleine Gewichtungsfaktoren $w_i$ unterdrückt. Beschleunigungen treten lediglich während der kurzen Momente auf, wenn ein Bewegungswechsel der Geschwindigkeit oder der Richtung vollzogen wird. Für die Fälle, in denen Beschleunigung mit der gleichen Größenordnung wie die Gravitationsbeschleunigung auftreten, kann $\Delta F_{absmax}$ klein genug gewählt werden, um Rechnungen zu unterdrücken und die Winkelwerte für kurze Perioden einzufrieren. Durch die Wahl einer längeren Verzögerungszeit (Delay Line) für das ausgleichende Hann-Filter und durch die Wahl eines höheren minimalen Schwellwertes $\Delta F_{axmin}$ können Korrekturen durch Bruchteile von Sekunden verzögert werden, so dass selbst schnelle Bewegung relativ stabil erscheinen.

Unter Zuhilfenahme des zusätzlich dargestellten gedrehten Bildes ist es möglich, sämtliche Aufgaben in kürzerer Zeit durchzuführen. Bewegungen des hybriden Instruments wurden mit der richtigen Ausrichtung auf der Anzeige des rotierten Bildes dargestellt, währenddessen auf dem Originalbild eine Bewegung in einer anderen Richtung zu sehen war. Insbesondere die Navigation in einem Winkelbereich zwischen 30 und 150˚ und zwischen 210 und 330˚ war extrem schwierig mittels eines Bildes ohne Rotationskorrektur (auch in Folge des FULCROM-Effekts). In allen Fällen können unter Nutzung von Ausführungsbeispielen gute Ergebnisse durch den Chirurgen festgestellt werden, die ein hybrides Instrument nutzten. Weitere Bildverbesserungen sind insbesondere dann möglich, wenn die Verzögerung der durchgeführten Drehung klein gewählt und die Bildqualität durch den angewandten USB-Frame Grabber möglichst hoch gewählt wird.

Ausführungsbeispiele der vorliegenden Erfindung können damit wie folgt zusammengefasst werden. Eine automatische Korrektur oder Umorientierung eines durch ein Endoskop erhaltenen Bildes in Echtzeit unterstützt den Betrachter in der Interpretation des rotierten Bildes, welches von einem flexiblen Videoskop erhalten wird. Dies trifft insbesondere auf Ärzte zu, die mit einer natürlichen Korrektur von Endoskopbildern in Bezug auf ein auf einen Patienten orientiertes kartesisches Koordinatensystem während der Operation vertraut sind. Andererseits sind Gastroenterologen bei der Betrachtung von luminalen Strukturen mit Endoskop-basierten Koordinatensysteme vertraut und können sich selbst dann orientieren, wenn das dargestellte Bild gedreht wird. Vorteilhafterweise können für NOTES-Systeme auch 2-Monitorsysteme verwendet werden, um Spezialisten beider Fachrichtungen (Chirurgen und Gastroenterologen) während ihres Eingriffes optimal zu unterstützen.

Somit lösen Ausführungsbeispielen das offene Problem des nicht vorhandenen stabilen Horizontes in der Endoskopie-Chirurgie (insbesondere für flexible Endoskope). Mit den beschriebenen Verfahren wird eine Bilddrehungskorrektur mittels kleiner tri-axialer MEMS-Inertialsensoren, die an der Endoskopspitze angebracht sind, durchgeführt. Die durchgeführte Drehrichtungskorrektur kann insbesondere für nicht starre Endoskopie-Chirurgie, wie beispielsweise NOTES verwendet werden. Dabei wird der die Richtung, aus der die Gravitation einwirkt, auf jede der drei orthogonalen Beschleunigungsachsen gemessen und nach einer Anfangskalibrierung und Filterung der drei gemessenen Werte werden Drehwinkel direkt bestimmt. Dies kann mit einer Abtastung mit einer Abtastrate durchgeführt werden, die oberhalb der Videowiederholfrequenz von 30 Hz liegt, wobei die Genauigkeit bei ungefähr 1˚ liegt. Die Drehrichtungskorrektur des Bildes kann in Echtzeit durch digitales Drehen des analogen Endoskopievideosignals durchgeführt werden. Die Verbesserung und der Nutzen wurden durch Tierversuche untersucht. Die Koordination der unterschiedlichen Instrumente und die Abschätzung des Gewebeverhaltens bezüglich von gravitationsbezogenen Deformationen und Bewegungen wurde mit einem stabilen Horizont in Endoskopiebildern als wesentlich intuitiver beurteilt.

Die Wirkung von Ausführungsbeispielen besteht somit darin, dass sich ein Inertialsensor 120 direkt am oder im Bildsensor 110 platzieren lässt. Das bietet den Vorteil, dass damit auch Orientierungen in Anwendungen gemessen und korrigiert werden können, deren Platzeinschränkungen dies bislang nicht zuließen. Somit wird bei Ausführungsbeispielen vorteilhafterweise der Inertialsensor in unmittelbarer Nähe zum Bildsensor platziert - also beim Endoskop in dessen Spitze und bei Kapsel im Allgemeinen. Das Inertialsensorsignal (Verkippungssignal) wird bei Ausführungsbeispielen gemeinsam mit dem Bildsensorsignal übertragen, sei es drahtgebunden im flexiblen Endoskop oder auch drahtlos in der Endoskopkapsel.

Ausführungsbeispiele der vorliegenden Erfindung werden aber nicht nur in der Chirurgie oder zur therapeutischen Zwecken genutzt, sondern können gleichermaßen auch in industriellen Anwendungen genutzt werden. So kann beispielsweise für die Untersuchung von Rohren oder Rohrsystemen oder anderen, dem bloßen Blick nicht zugänglichen Hohlräumen, ein flexibles Endoskop genutzt werden, wobei es auch bei diesen Anwendungen wichtig ist, einen Horizont bei der Betrachtung der entsprechenden Bilder zu definieren bzw. anzupassen. Derlei technische Einsatzgebiete umfassen beispielsweise den Bauschutz zur Überprüfung von Isolierungen von Altbauten z.B., aber auch die Denkmalpflege, da große Denkmäler oftmals hohl sein können und mittels Endoskopie auf etwaige korrosive Vorgänge geprüft werden können. Weitere Anwendungen finden sich in der Automobilindustrie, wobei die Endoskope hauptsächlich zur Prüfung von Hohlraumversiegelungen und Motoren z.B. bei Verschleißuntersuchungen eingesetzt werden. Ebenfalls werden Endoskope in der Schiffsindustrie zur Untersuchung von Motoren oder in Industrieanlagen, wie beispielsweise Kraftwerke, Rohrschweißnähte und Ähnlichem genutzt. Im Sanitärbereich finden Endoskope beispielsweise Anwendungen bei der Untersuchung defekter Leitungen oder auch in der Luftfahrt zur Untersuchung von Turbinen. Besonders in der Luftfahrt wird die Endoskopie immer öfter für die Wartung z.B. von Flugtriebwerken eingesetzt, wobei unter Zuhilfenahme eines Arbeitskanals und von Mikrowerkzeugen auch kleinere Reparaturen an Triebwerksschaufeln durchgeführt werden können. So etablierte sich beispielsweise in diesem Bereich der Begriff Boroskopie. Insbesondere auch in diesen technischen Anwendungen werden in der Regel flexible Endoskope genutzt, da ein starres Teleskop in den seltensten Fällen in verwinkelte Hohlräume eingeführt werden kann. Wie bereits gesagt, kann das flexible Endoskop ferner Arbeitskanäle aufweisen, durch die flexible Arbeitswerkzeuge genutzt werden können. Die flexiblen Arbeitswerkzeuge umfassen beispielsweise Greif-oder Schneidwerkzeuge z.B. zwecks Gewinnung von Gewebeproben, Kanülen zur Injektion oder auch Drahtelektroden zur Anwendung eines elektrischen Stroms.

**Patentansprüche**

1. Endoskop (100) mit einem Bildsensor (110) und einem Inertialsensor (120) am distalen Ende (105) desselben, wobei der Inertialsensor (120) ausgebildet ist, um eine Verkippung des Bildsensors (110) zu erfassen, wobei das Endoskop ferner eine Steuereinrichtung (130) aufweist, wobei der Bildsensor (110) ein Array von Bildpixel umfassend ausgebildet ist, um Bilddaten zu erzeugen, und der Inertialsensor (120) ausgebildet ist, um ein die Verkippung repräsentierendes Verkippungssignal zu erzeugen, **dadurch gekennzeichnet dass** die Steuereinrichtung (130) ausgebildet ist, um das Verkippungssignal mit den Bilddaten unter Ersetzung eines Teils der Bilddaten durch das Verkippungssignal zu einem Kombinationssignal zu kombinieren.

2. Endoskop (100) nach Anspruch 1, das eine Einrichtung zum Übertragen des Kombinationssignals von der Steuereinrichtung (130) zu einem proximalen Ende des Endoskops (100) aufweist.

3. Endoskop (100) nach Anspruch 1 oder Anspruch 2, bei dem die Steuereinrichtung (130) ausgebildet ist, um in den Bilddaten einen Pixelwert eines Pixels durch das Verkippungssignal zu ersetzen.

4. Endoskop (100) nach Anspruch 3, bei dem die Steuereinrichtung (130) so ausgebildet ist, dass das Pixel ein Randpixel des Arrays von Pixels ist.

5. Endoskop (100) nach einem der Ansprüche 1 bis 4, bei dem der Bildsensor (110) einen Analog/Digital-Wandler aufweist, der ausgebildet ist, um Pixelwerte der Pixel des Arrays von Pixeln zu digitalisieren, wobei der Inertialsensor (120) und die Steuereinrichtung (130) so ausgebildet sind, dass das Verkippungssignal in analoger Form von dem Inertialsensor (120) ausgegeben und anstelle eines Pixelwertes eines Pixels des Array von Pixeln durch den Analog/Digital-Wandler des Bildsensors (110) digitalisiert wird.

6. Endoskop (100) nach einem der Ansprüche 1 bis 4, bei dem die Steuereinrichtung (130) und der Inertialsensor (120) jeweils einen eigenen Analog/Digital-Wandler aufweisen, um die Bilddaten und das Verkippungssignal zu

**EP 2 323 541 B1**

digitalisieren, und bei dem die Steuereinrichtung (130) ferner ausgebildet ist, um Bits der digitalen Bilddaten durch Bits des digitalen Verkippungssignals zu ersetzen.

7. Endoskop (100) nach Anspruch 6, bei dem die Steuereinheit (130) ausgebildet ist, um einen niedrigwertigen Anteil der Bits eines Pixels durch Bits des digitalen Verkippungssignals zu ersetzen und einen höherwertigen Anteil der Bits der Pixel in das Kombinationssignal zu übernehmen.

8. Endoskop (100) nach einem der vorhergehenden Ansprüche, bei dem der Bildsensor (110) ausgebildet ist, ein Farbbild mit drei Farbkomponenten zu erfassen und bei dem Inertialsensor (120) einen ersten Inertialsensor (120a), einen zweiten Inertialsensor (120b) und einen dritten Inertialsensor (120c) aufweist, wobei der erste, zweite und dritte Inertialsensor (120a, 120b, 120c) so ausgebildet sind, so dass jeder derselben ein anderes Verkippungssignal für Drehungen um andere räumliche Drehachsen erfasst.

9. Endoskop (100) nach einem der vorhergehenden Ansprüche, bei dem das Endoskop (100) eine flexibles Endoskop ist.

10. Endoskop (100) nach einem der Ansprüche 1 bis 9, bei dem der Bildsensor (110) und der Inertialsensor (120) auf einem gemeinsamen Chip integriert sind.

11. Bildaufnahmevorrichtung mit:

   einem Bildsensor (110) zum Erzeugen von Bilddaten;
   einem Inertialsensor (120) zur Erfassung einer Verkippung der Bildaufnahmevorrichtung, um ein Verkippungssignal zu erhalten; und **dadurch gekennzeichnet dass** die Bildaufnahme vorrichtung
   eine Steuereinrichtung (130) zum Kombinieren des Verkippungssignals und der Bilddaten zu einem Kombinationssignal unter Ersetzung von Teilen der Bilddaten durch das Verkippungssignal enthält.

12. Bildaufnahmevorrichtung nach Anspruch 11, wobei die Bildaufnahmevorrichtung länglich ist und ein erstes und ein zweites Ende aufweist, wobei der Bildsensor (110), der Inertialsensor (120) und die Steuereinheit (130) an einem ersten Ende angeordnet sind, und die Steuereinrichtung (130) ausgebildet ist, das Kombinationssignal über eine elektrische Übertragungsleitung (135) oder drahtlos zu dem zweiten Ende zu übertragen.

13. Bildaufnahmevorrichtung nach Anspruch 11 oder Anspruch 11, bei der der Inertialsensor (120) ausgebildet ist, um die Verkippung zeitlich abzutasten, um eine Sequenz von Verkippungswerte zu erhalten,
und bei der die Steuereinrichtung (130) ein Filter und/oder eine Schwellwerteinheit aufweist, wobei das Filter ausgebildet ist, um eine Glättung der zeitlichen Sequenz von Verkippungswerten durchzuführen, und bei der die Schwellwerteinheit ausgebildet ist, um aus der zeitlichen Sequenz der Verkippungswerte Werte zu eliminieren, die unterhalb eines vorgegebenen Schwellwerts liegen.

14. Verfahren zur Aufnahme eines Bildes, mit folgenden Schritten:

   Erzeugen von Bilddaten durch einen Bildsensor (110);
   Erfassen einer Verkippung des Bildsensors durch einen Inertialsensor (120) und Bereitstellen eines Verkippungssignals; und **gekennzeichnet durch** das
   Kombinieren des Verkippungssignals mit den Bilddaten zu einem erweiterten Datenstrom unter Ersetzung von Teilen der Bilddaten **durch** das Verkippungssignal.

**Claims**

1. Endoscope (100) comprising an image sensor (110) and an inertial sensor (120) at the distal end (105) of same, the inertial sensor (120) being configured to sense tilting of the image sensor (110), the endoscope further comprising a control means (130), the image sensor (110) being configured to comprise an array of image pixels to produce image data, and the inertial sensor (120) being configured to produce a tilting signal, **characterized in that** the control means (130) is configured to combine into a combination signal the tilting signal with the image data while replacing some of the image data by the tilting signal.

2. Endoscope (100) as claimed in claim 1, comprising a means for transmitting the combination signal from the control means (130) to a proximal end of the endoscope (100).

3. Endoscope (100) as claimed in claim 1 or claim 2, wherein the control means (130) is configured to replace, in the image data, a pixel value of a pixel by the tilting signal.

4. Endoscope (100) as claimed in claim 3, wherein the control means (130) is configured such that the pixel is an edge pixel of the array of pixels.

5. Endoscope (100) as claimed in any of claims 1 to 4, wherein the image sensor (110) comprises an analog-to-digital converter configured to digitize pixel values of the pixels of the array of pixels,
the inertial sensor (120) and the control means (130) being configured such that the tilting signal is output by the inertial sensor (120) in an analog form and is digitized by the analog-to-digital converter of the image sensor (110) instead of a pixel value of a pixel of the array of pixels.

6. Endoscope (100) as claimed in any of claims 1 to 4, wherein the control means (130) and the initial sensor (120) each comprise an analog-to-digital converter of their own so as to digitize the image data and the tilting signal, and wherein the control means (130) is further configured to replace bits of the digital image data by bits of the digital tilting signal.

7. Endoscope (100) as claimed in claim 6, wherein the control unit (130) is configured to replace a low-significance portion of the bits of a pixel by bits of the digital tilting signal and to carry over a higher-significance portion of the bits of the pixels into the combination signal.

8. Endoscope (100) as claimed in any of the previous claims, wherein the image sensor (110) is configured to sense a color image having three color components, and wherein the inertial sensor (120) comprises a first inertial sensor (120a), a second inertial sensor (120b), and a third inertial sensor (120c), the first, second and third inertial sensors (120a, 120b, 120c) being configured such that each of same senses a different tilting signal for rotations about other spatial axes of rotation.

9. Endoscope (100) as claimed in any of the previous claims, wherein the endoscope (100) is a flexible endoscope.

10. Endoscope (100) as claimed in any of claims 1 to 9, wherein the image sensor (110) and the inertial sensor (120) are integrated on a shared chip.

11. Image-capturing device comprising:

   an image sensor (110) for producing image data;
   an inertial sensor (120) for sensing any tilting of the image-capturing device so as to obtain a tilting signal; and

   **characterized in that** the image-capturing device contains
   a control means (130) for combining the tilting signal and the image data into a combination signal while replacing some of the image data by the tilting signal.

12. Image-capturing device as claimed in claim 11, the image-capturing device being elongated and comprising a first and a second end, the image sensor (110), the inertial sensor (120) and the control unit (130) being arranged at a first end, and the control means (130) being configured to transmit the combination signal to the second end via an electrical transmission line (135) or in a wireless manner.

13. Image-capturing device as claimed in claim 11 or claim 11, wherein the inertial sensor (120) is configured to time-sample the tilting so as to obtain a sequence of tilting values,
and wherein the control means (130) comprises a filter and/or a threshold-value unit, the filter being configured to perform smoothening of the temporal sequence of tilting values, and wherein the threshold-value unit is configured to eliminate, from the temporal sequence of the tilting values, such values which fall below a predefined threshold value.

14. Method of capturing an image, comprising:

   producing image data on the part of an image sensor (110);

sensing tilting of the image sensor on the part of an inertial sensor (120), and providing a tilting signal; and

**characterized by** the
combining of the tilting signal with the image data into an expanded data stream while replacing some of the image data by the tilting signal.

**Revendications**

1. Endoscope (100) avec un capteur d'image (110) et un capteur d'inertie (120) à son extrémité distale (105), dans lequel le capteur d'inertie (120) est réalisé pour saisir un basculement du capteur d'image (110), dans lequel l'endoscope présente par ailleurs un moyen de commande (130), dans lequel le capteur d'image (110) comportant un réseau de pixels d'image est réalisé pour générer des données d'image, et le capteur d'inertie (120) est réalisé pour générer un signal de basculement représentant le basculement, **caractérisé par le fait que** le moyen de commande (130) est réalisé pour combiner le signal de basculement avec les données d'image en remplaçant une partie des données d'image par le signal de basculement, pour obtenir un signal de combinaison.

2. Endoscope (100) selon la revendication 1, présentant un moyen destiné à transmettre le signal de combinaison du moyen de commande (130) à une extrémité proximale de l'endoscope (100).

3. Endoscope (100) selon la revendication 1 ou la revendication 2, dans lequel le moyen de commande (130) est réalisé pour remplacer, dans les données d'image, une valeur d'un pixel par le signal de basculement.

4. Endoscope (100) selon la revendication 3, dans lequel le moyen de commande (130) est réalisé de sorte que le pixel soit un pixel de bordure du réseau de pixels.

5. Endoscope (100) selon l'une des revendications 1 à 4, dans lequel le capteur d'image (110) présente un convertisseur analogique/ numérique qui et réalisé pour numériser les valeurs des pixels des réseaux de pixels, le capteur d'inertie (120) et le moyen de commande (130) étant réalisés de sorte que le signal de basculement soit sorti de forme analogique par le capteur d'inertie (120) et soit numérisé au lieu d'une valeur d'un pixel du réseau de pixels par le convertisseur analogique/numérique du capteur d'image (110).

6. Endoscope (100) selon l'une des revendications 1 à 4, dans lequel le moyen de commande (130) et le capteur d'inertie (120) présentent, chacun, un convertisseur analogique/numérique qui leur est propre, pour numériser les données d'image et le signal de basculement, et dans lequel le moyen de commande (130) est réalisé par ailleurs pour remplacer des bits des données d'image numériques par des bits du signal de basculement numérique.

7. Endoscope (100) selon la revendication 6, dans lequel l'unité de commande (130) est réalisée pour remplacer une part de faible valeur des bits d'un pixel par des bits du signal de basculement numérique et pour reprendre une part de valeur élevée des bits des pixels dans le signal de combinaison.

8. Endoscope (100) selon l'une des revendications précédentes, dans lequel le capteur d'image (110) est réalisé pour saisir une image de couleur à trois composantes de couleur et dans lequel le capteur d'inertie (120) présente un premier capteur d'inertie (120a), un deuxième capteur d'inertie (120b) et un troisième capteur d'inertie (120c), le premier, le deuxième et le troisième capteur d'inertie (120a, 120b, 120c) étant réalisés de sorte que chacun d'eux saisisse un autre signal de basculement pour des rotations autour d'autres axes de rotation spatiaux.

9. Endoscope (100) selon l'une des revendications précédentes, dans lequel l'endoscope (100) est un endoscope flexible.

10. Endoscope (100) selon l'une des revendications 1 à 9, dans lequel le capteur d'image (110) et le capteur d'inertie (120) sont intégrés sur une puce commune.

11. Dispositif de prise de vue, avec:

    un capteur d'image (110) destiné à générer des données d'image;
    un capteur d'inertie (120) destiné à saisir un basculement du dispositif de prise de vue, pour obtenir un signal de basculement; et

**caractérisé par le fait que** le dispositif de prise de vue contient
un moyen de commande (130) destiné à combiner le signal de basculement et les données d'image, pour obtenir un signal de combinaison en remplaçant des parties des données d'image par le signal de basculement.

12. Dispositif de prise de vue selon la revendication 11, dans lequel le dispositif de prise de vue est allongé et présente une première et une deuxième extrémité, dans lequel le capteur d'image (110), le capteur d'inertie (120) et l'unité de commande (130) sont disposés à une première extrémité, et le moyen de commande (130) est réalisé pour transmettre le signal de combinaison via une ligne de transmission électrique (135) ou sans fil à la deuxième extrémité.

13. Dispositif de prise de vue selon la revendication 11 ou la revendication 11, dans lequel le capteur d'inertie (120) est réalisé pour balayer le basculement dans le temps, pour obtenir une séquence de valeurs de basculement, et dans lequel le moyen de commande (130) présente un filtre et/ou une unité de valeur de seuil, le filtre étant réalisé pour effectuer un aplanissement de la séquence temporelle de valeurs de basculement, et dans lequel l'unité de valeur de seuil est réalisée pour éliminer de la séquence temporelle de valeurs de basculement les valeurs qui se situent au-dessous d'une valeur de seuil prédéterminée.

14. Procédé de prise de vue d'une image, aux étapes suivantes consistant à:

   générer des données d'image par un capteur d'image (110);
   saisir un basculement du capteur d'image du capteur d'image par un capteur d'inertie (120) et préparer un signal de basculement; et

   **caractérisé par** le fait de
   combiner le signal de basculement avec les données d'image, pour obtenir un flux de données élargi en remplaçant des parties des données d'image par le signal de basculement.

FIG 1

FIG 2

FIG 3

EP 2 323 541 B1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7211042 B2
- US 20080159653 A1
- US 20050020883 A1